Europäisches Patentamt

(19) European Patent Office

Office européen des brevets

(11) Numéro de publication: **0 218 492**
**B1**

(12) **FASCICULE DE BREVET EUROPEEN**

(45) Date de publication du fascicule du brevet:
08.11.89

(21) Numéro de dépôt: 86401871.8

(22) Date de dépôt: 26.08.86

(51) Int. Cl.⁴: **A61B 17/58, A61B 17/16**

(54) Sonde de percage pour clou intramédullaire et clou intramédullaire approprié.

(30) Priorité: **27.08.85 FR 8512777**
**13.08.86 FR 8611720**

(43) Date de publication de la demande:
**15.04.87 Bulletin 87/16**

(45) Mention de la délivrance du brevet:
**08.11.89 Bulletin 89/45**

(84) Etats contractants désignés:
**AT BE CH DE GB IT LI SE**

(56) Documents cités:
**EP-A- 0 135 804**
**FR-A- 1 146 824**
**FR-A- 2 289 155**
**FR-A- 2 344 267**
**SU-A- 184 393**
**US-A- 2 878 809**
**US-A- 3 439 671**

(73) Titulaire: **ZIMMER S.A. Société dite:, 68-70 rue du Général Malleret-Joinville B.P. 104, F-94401 Vitry-sur-Seine Cédex(FR)**

(72) Inventeur: **Tanguy, Christian, 19 rue Gambetta, F-94600 Choisy Le Roi(FR)**

(74) Mandataire: **Hirsch, Marc-Roger, Cabinet Hirsch 34 rue de Bassano, F-75008 Paris(FR)**

## Description

La présente invention concerne une sonde de guidage, de positionnement et de perçage destinée à être utilisée pour la fixation d'un clou intramédullaire préalablement mis en place dans la cavité médullaire d'un os endommagé. Une sonde de ce type est connue de la demande de brevet européen EP-A2 0 135 804. Elle concerne également un clou intramédullaire propre à être fixé en place à l'aide de cette sonde.

L'invention vise plus particulièrement un clou intramédullaire à verrouillage, c'est-à-dire appelé à être immobilisé par rapport à l'os par au moins une vis traversant deux trous de vis opposés et alignés préalablement ménagés dans le clou et traversant également les corticales adjacentes de part et d'autre du clou, cependant que l'outillage de fixation précité est destiné à permettre la mise en place d'une telle vis.

Le clou intramédullaire considéré ici est du type constitué par un élément rigide, notamment métallique, quasi-tubulaire qui présente une section transversale non-circulaire constante au moins sur la majeure partie de la longueur du clou. En règle générale, les clous de ce genre ne présentent pas une paroi périphérique continue, mais définissent une fente longitudinale s'étendant sur toute la longueur du clou.

La méthode universellement employée jusqu'à présent pour la mise en place, ou implantation, d'un tel clou intramédullaire, notamment en position distale, consiste essentiellement à percer, à une extrémité dite proximale de l'os à soigner, une ouverture donnant accès longitudinalement à la cavité médullaire, à introduire le clou dans celle-ci à travers cette ouverture, à le déplacer longitudinalement dans la cavité jusqu'à ce qu'il ait atteint la profondeur de pénétration voulue, à repérer l'emplacement et la position des trous de vis du clou, à percer dans les deux corticales opposées adjacentes auxdits trous de vis des trous de fixation alignés avec précision sur les trous de vis, à introduire une vis dans ces trous de vis et de fixation et à l'immobiliser par vissage dans la corticale recevant la pointe (ou extrémité avant) de la vis, le trou de fixation pratiqué dans cette corticale présentant à cet effet un diamètre inférieur au diamètre du sommet du filetage de la vis.

Or, cette méthode, qui est la seule que l'on puisse appliquer en utilisant l'équipement chirurgical existant, présente un inconvénient grave en ce que le repérage des endroits où l'on doit percer l'os transversalement afin d'y pratiquer les trous de fixation précités en alignement précis avec les trous de vis du clou préalablement mis en place, est une opération très délicate. Dans la plupart des cas, le chirurgien a recours, à cet effet, à la radioscopie, utilisant des amplificateurs de brillance, des cadres de visée, etc. Ceci complique et allonge le travail du chirurgien, tout en prolongeant la durée pendant laquelle le patient est maintenu sous anesthésie, et surtout expose le chirurgien aussi bien que le patient à une dose indésirable de rayonnement X.

La présente invention a pour but de créer un dispositif, notamment une sonde de guidage, de positionnement et de perçage permettant d'éliminer les inconvénients ci-dessus lors de son utilisation pour la fixation d'un clou intramédullaire approprié, grâce à la possibilité qu'il offre d'appliquer une méthode nouvelle de fixation sans avoir à recourir à des procédures fastidieuses telles que radioscopie ou analogues.

Afin d'atteindre ce but, la sonde de perçage destinée à venir se loger à l'intérieur d'un clou tubulaire intramédullaire à partir d'une extrémité accessible du clou pour forer un passage ou un alésage dans la paroi corticale d'un os qui porte le clou est caractérisée en ce qu'elle comporte un bloc de guidage et de positionnement qui est guidé et mobile axialement dans la cavité interne du clou et qui supporte une gaine traversée intérieurement par un arbre flexible portant, à son extrémité distale, une tête de perçage et à son extrémité proximale, des moyens d'accouplement à un moteur rotatif et en ce que ladite gaine débouche à son extrémité proximale axialement à partir de la cavité intérieure du clou et respectivement à son extrémité distale, latéralement en direction de la paroi intérieure du clou par un orifice distal situé en face d'un trou de vis ménagé dans la paroi du clou, ledit bloc comportant en outre des moyens de positionnement aptes à coopérer avec des moyens de position nement complémentaires du clou intramédullaire de manière à positionner la sortie proximale de la gaine en face du trou de vis correspondant.

La sonde de perçage peut comporter un arbre flexible monté à rotation et axialement déplaçable dans une gaine flexible présentant un diamètre extérieur sensiblement plus petit que la dimension minimum de la section transversale de la cavité définie dans le clou intramédullaire, ledit arbre flexible portant à une extrémité distale une tête de perçage et à son extrémité proximale des moyens d'accouplement à un moteur rotatif, cependant que ladite gaine est solidarisée à son extrémité distale d'un bloc de guidage et de positionnement pourvu d'un passage interne qui est raccordé à l'alésage de ladite gaine et présente une section transversale identique à celle de cet alésage, ledit passage comportant une première partie de passage alignée sur l'alésage de la gaine et une seconde partie de passage formant avec ladite première partie un angle obtus et débouchant à la surface dudit bloc de guidage par un orifice susceptible d'être aligné sur un trou de vis d'entrée ménagé dans la paroi du clou intramédullaire intéressé, ledit bloc comportant en outre des moyens de positionnement appelés à coopérer avec des moyens de positionnement complémentaires du clou intramédullaire, le profil extérieur de ce bloc de guidage étant conformé au profil intérieur du clou intramédullaire de manière à pouvoir être introduit dans l'extrémité proximale de celui-ci et être déplacé longitudinalement par coulissement dans ledit clou, tout en étant immobilisé en rotation.

Avantageusement, lesdits moyens de positionnement du bloc de guidage comportent une bille montée de manière déplaçable dans un logement ménagé dans ledit bloc selon un axe sensiblement transversal à la direction de coulissement dudit bloc dans le clou et décalé angulairement par rapport audit orifice du passage, cette bille étant sollicitée par un ressort placé dans ledit logement et tendant à maintenir la bille dans une position dans laquelle elle dépasse de la surface extérieure dudit bloc de manière à pouvoir coopérer avec lesdits moyens de positionnement complémentaires du clou qui sont constitués par une cavité ménagée dans la paroi du clou dans une position déterminée par rapport audit trou de vis d'entrée du clou, l'agencement étant tel que l'engagement de la bille dans la cavité de positionnement se produise lorsque l'orifice du passage est aligné sur le trou de vis d'entrée du clou.

Dans un mode de réalisation préféré, la cavité de positionnement est constituée par un trou de positionnement traversant la paroi du clou.

Le logement ainsi que l'orifice du passage sont disposés de préférence dans des zones dudit bloc qui sont en contact direct avec la paroi interne du clou lorsque ledit bloc est introduit dans ce dernier.

Avantageusement, le bloc de guidage est pourvu d'une tige de manipulation s'étendant dans la même direction générale que la gaine flexible et pourvue d'une poignée de préhension qui dépasse de l'extrémité proximale dudit clou pour toute position longitudinale dudit bloc de guidage à l'intérieur du clou.

Le clou intramédullaire creux selon l'invention qui comporte une cavité interne propre à être utilisé en combinaison avec la sonde de guidage définie ci-dessus présente un profil transversal non-circulaire constant sur la majeure partie de sa longueur et est pourvu, au moins dans la partie distale de sa paroi, d'au moins un trou de vis d'entrée et d'au moins un trou de vis de sortie aligné selon un même axe commun sur le trou de vis d'entrée, ce clou étant caractérisé essentiellement en ce que ledit axe commun d'alignement desdits trous de vis d'entrée et de sortie est incliné par rapport à l'axe médian longitudinal dudit clou sous un angle égal à l'angle d'inclinaison de la seconde partie du passage dudit bloc de guidage de ladite sonde de guidage par rapport à la première partie de ce passage, et en ce qu'une cavité de positionnement appelée à coopérer avec la bille de positionnement dudit bloc est ménagée dans la paroi du clou, dans une partie de celle-ci qui est appelée à entrer en contact direct avec la surface dudit bloc lorsque celui-ci est amené, par coulissement dans le clou, dans la zone de cette partie de paroi.

De préférence, ladite cavité de positionnement est constituée par un trou de positionnement traversant la paroi du clou.

L'axe du trou de positionnement précité est situé dans le même plan que l'axe commun desdits trous de vis d'entrée et de sortie.

Dans ce cas, le diamètre du trou de positionnement est, de préférence, sensiblement plus grand que celui du trou de vis de sortie.

Dans une variante, cet axe du trou de positionnement est situé dans un plan décalé angulairement par rapport au plan contenant l'axe commun des trous de vis d'entrée et de sortie.

La section transversale du clou intramédullaire creux présente une pluralité de lobes, cependant que le bloc de guidage présente un profil extérieur épousant au moins un desdits lobes.

De préférence, les lobes définis par la section transversale du clou sont au nombre de trois, et le profil extérieur dudit bloc de guidage épouse deux de ces lobes.

Le bloc de guidage est pourvu d'une tige rigide de manipulation dont l'extrémité dépasse, pour toute position dudit bloc dans le clou intramédullaire, de l'extrémité proximale de celui-ci.

Il ressort de ce qui précède que, grâce à la présente invention, il est désormais possible de procéder à la mise en place des vis de fixation d'un clou intramédullaire dans la cavité médullaire intéressée en perçant les corticales de l'intérieur aux endroits voulus, l'outil de perçage étant positionner avec une précision totale, sans que l'on ait à recourir aux procédures classiques de repérage, de positionnement et perçage sous contrôle radioscopique.

L'invention sera décrite ci-dessous de manière plus détaillée, notamment en référence aux figures annexées, étant bien entendu que cette description et ces figures sont données à titre d'illustration seulement, mais non pas à titre de limitation.

- la figure 1 représente schématiquement, en coupe longitudinale, un mode de réalisation de l'équipement chirurgical selon l'invention;
- la figure 2 montre d'une part, en A, également en coupe longitudinale, le bloc de guidage et, d'autre part, en B, une coupe transversale du même bloc disposé à l'intérieur du clou intramédullaire.
- la figure 3 est une coupe longitudinale d'une partie du clou intramédullaire selon l'invention et montre plus particulièrement différentes dispositions des trous de vis et de positionnement;
- la figure 4 illustre sous forme de coupes schématiques longitudinales A à F différentes étapes successives de l'utilisation de l'équipement selon l'invention pour la consolidation d'un fémur;
- la figure 5 montre, en coupe longitudinale, un clou intramédullaire selon l'invention mis en place dans un fémur dans lequel ont été pratiqué les trous appelés à recevoir une vis;
- la figure 6 est une coupe analogue à celle de la figure 5 qui montre un clou intramédullaire selon l'invention mis en place dans un fémur où il est fixé par deux vis.
- la figure 7 représente schématiquement, en coupe longitudinale, un autre mode de réalisation de la sonde de perçage selon l'invention, mise en place dans un clou intramédullaire pour y forer radialement un os;
- la figure 8 montre, d'une part en A, également en coupe longitudinale et à plus grande échelle, le bloc de guidage de la sonde et, d'autre part en B, une coupe transversale à plus grande échelle, selon la ligne IIB de la figure 7, du même bloc disposé à l'intérieur du clou intramédullaire;
- la figure 9 est une coupe longitudinale d'une partie du clou intramédullaire selon l'invention et montre, plus particulièrement, différentes dispositions des trous de vis et de positionnement ménagés dans ce clou.

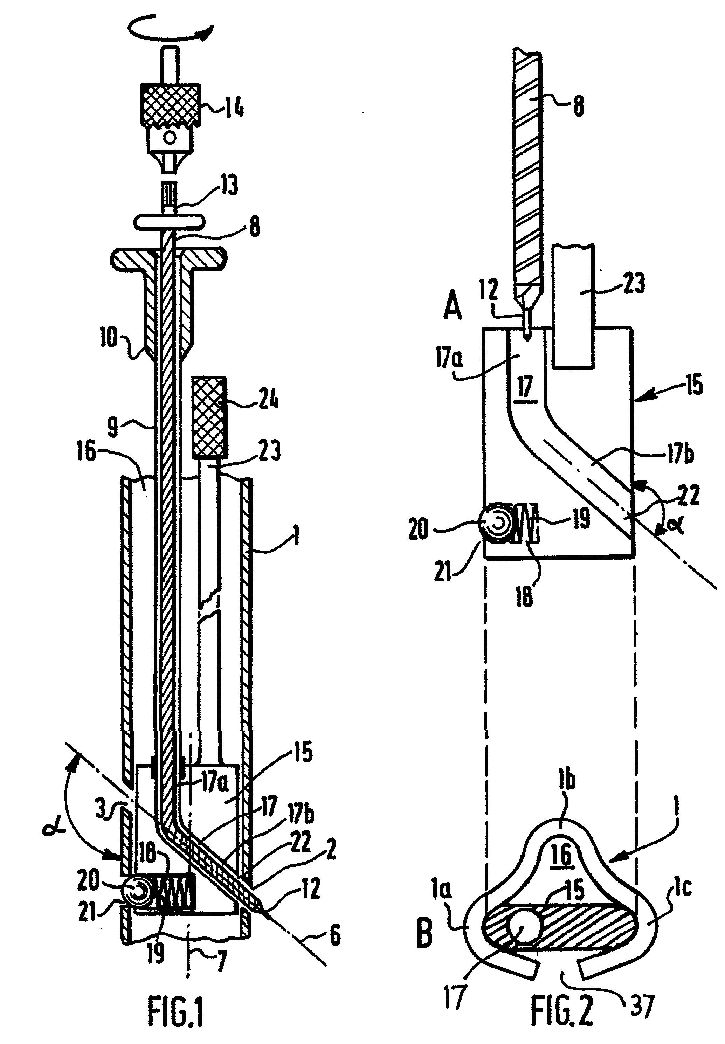

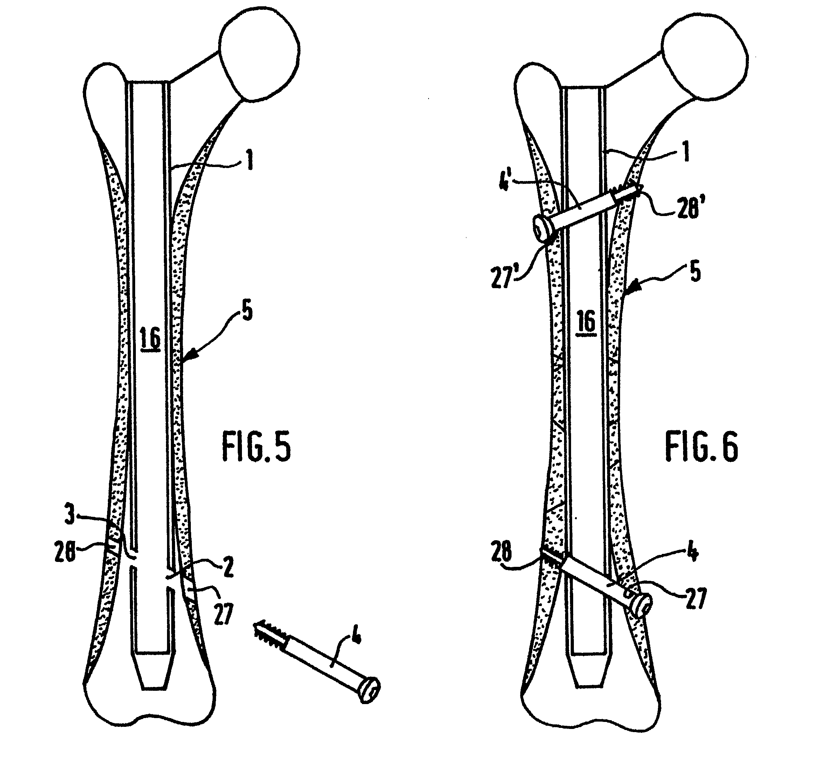

L'équipement selon l'invention comprend un clou intramédullaire 1, représenté en coupe longitudinale sur la figure 1. Dans le présent mode de réalisation, la section transversale, non circulaire, du clou 1 définit trois lobes 1a, 1b, 1c disposés sensiblement selon un triangle isocèle, ainsi que le montre la partie B de la figure 2. Le clou 1 est pourvu de deux trous de vis 2 et 3 ménagés respectivement dans les lobes 1c et 1a et appelés à recevoir une vis 4 (figure 5) de verrouillage du clou dans la cavité médullaire d'un os, par exemple d'un fémur 5. Cette vis est susceptible d'être introduite par le trou 2, ou trou d'entrée, et de traverser ensuite, par son extrémité avant (ou pointe) le trou 3, ou trou de sortie. Pour des raisons qui ressortiront de la suite de la description, les trous de vis d'entrée et de sortie 2, 3, qui sont alignés l'un sur l'autre, sont disposés selon une axe géométrique commun 6 qui forme un angle obtus alpha avec l'axe géométrique longitudinal médian 7 du clou 1 et, par conséquent, avec une génératrice de la paroi de celui-ci. Il convient de noter que le profil -notamment le profil intérieur- du clou creux 1 est constant sur toute la longueur d'une partie sensiblement rectiligne du clou, et que la présente description se réfère à cette partie rectiligne à profil constant. Le clou selon le présent mode de réalisation comporte, d'une manière bien connue en soi, une fente longitudinale 37 (figure 2) s'étendant sur pratiquement toute la longueur du clou.

L'equipement selon l'invention comprend, par ailleurs, un arbre flexible 8 monté, d'une manière connue en soi, à rotation dans une gaine tubulaire flexible 9 pourvue à l'une de ses extrémités (appelée ci-après "extrémité proximale") une poignée tubulaire 10 solidarisée de ladite gaine et appelée à faciliter l'introduction de l'arbre flexible 8 dans celle-ci, ainsi que son retrait.

L'arbre flexible 8 porte à l'une de ses extrémités (appelée ci-après "extrémité distale") un outil de forage ou perçage, tel qu'une mèche 12 du type couramment utilisé en chirurgie pour le perçage des os. A son autre extrémité, ou extrémité proximale, l'arbre flexible 8 est muni de moyens d'accouplement 13 à un moteur rotatif 14.

La gaine 9 est fixée par son extrémité opposée à la poignée 10, ou extrémité distale, sur un bloc de guidage 15 présentant un contour tel qu'il puisse être introduit dans la cavité définie par le clou 1 et qu'il puisse y être déplacé à coulissement dans la direction de l'axe 7 du clou, tout en étant immobilisé en rotation par rapport à celui-ci. A cet effet, le bloc 15 épouse, dans le présent exemple, le profil intérieur concave des deux lobes opposés 1a et 1c du clou 1, ainsi qu'il ressort notamment de la coupe transversale représentée en B sur la figure 2.

Le bloc de guidage 15 est pourvu d'un passage interne 17 dont la section transversale correspond à celle de l'alésage ou passage défini à l'intérieur de la gaine 9 et servant de logement au flexible 8. La connexion entre la gaine 9 et le bloc 15 est telle que le passage 17 soit raccordé à l'alésage ou logement précité, ainsi qu'il ressort de la figure 1. Une première partie, ou partie d'entrée 17a, du passage 17 est alignée sur la partie terminale distale de la gaine 9 et s'étend donc sensiblement parallèlement à l'axe médian longitudinal 7 du clou 1 lorsque le bloc 15 est placé dans la cavité 16 du clou, cependant qu'une seconde partie, ou partie distale 17b dudit passage qui aboutit à un orifice de sortie 22, forme avec la partie proximale 17a (et, par conséquent, avec l'axe 7 ou avec une génératrice du clou 1) un angle obtus égal à l'angle alpha mentionné ci-dessus. La valeur de cet angle est choisi en fonction, notamment, des caractéristiques de la transmission flexible constituée par l'arbre 8 et la gaine 9, c'est-à-dire qu'elle ne doit pas être inférieure à une valeur limite à partir de laquelle une transmission satisfaisante du couple de rotation entre le moteur 14 et la mèche 12 ne serait plus assurée.

Le bloc de guidage 15 est pourvu d'un logement cylindrique bor gne 18 s'étendant perpendiculairement à l'axe 7 (en référence au bloc 15 introduit dans le clou 1) et renfermant un ressort hélicoïdal 19 coaxial audit logement, qui sollicite vers l'extérieur du bloc 15 une bille de positionnement 20 capable de se déplacer dans le logement 18 dans la direction de l'axe géométrique de celui-ci, et de dépasser, sous l'action du ressort 20, de l'orifice de logement sans pouvoir toutefois s'en échapper. La bille coopère par encliquetage avec une cavité de positionnement formée dans le présent exemple par un trou 21 ménagé dans la paroi du clou 1 dans le lobe opposé à celui qui présente le trou de vis d'entrée 2. L'emplacement du trou de positionnement 21 est choisi tel - compte tenu de l'agencement du bloc de guidage que la bille 20 pénètre partiellement dans le trou 21 lorsque l'orifice de sortie 22 du passage 17 se trouve aligné sur le trou de vis d'entrée 2 du clou 1. Dans le mode de réalisation représenté, le trou de positionnement 21 et le trou de vis de sortie 3 ont leurs centres respectifs placés sur une même génératrice de la paroi du clou 1. Par conséquent, afin d'éviter des erreurs de positionnement, on donne au trou de positionnement 21 un diamètre sensiblement plus grand que celui du trou de vis de sortie 3, ce qui entraîne l'utilisation d'une bille 20 de diamètre relativement grand, de sorte que celle-ci ne risque pas, lors de la mise en place du bloc 15, de produire un effet d'encliquetage dans le trou de vis 3, qui pourrait être interprété à tort comme indiquant que le bloc 15 a atteint la position voulue dans la cavité 16 du clou.

Afin d'en faciliter la manipulation, le bloc 15 est doté d'une tige rigide 23 s'étendant dans la même direction générale que la gaine 9 et muni, à son extrémité libre, d'une poignée de préhension 24 qui dépasse de l'extrémité proximale du clou 1 pour toute position du bloc 15 dans celui-ci.

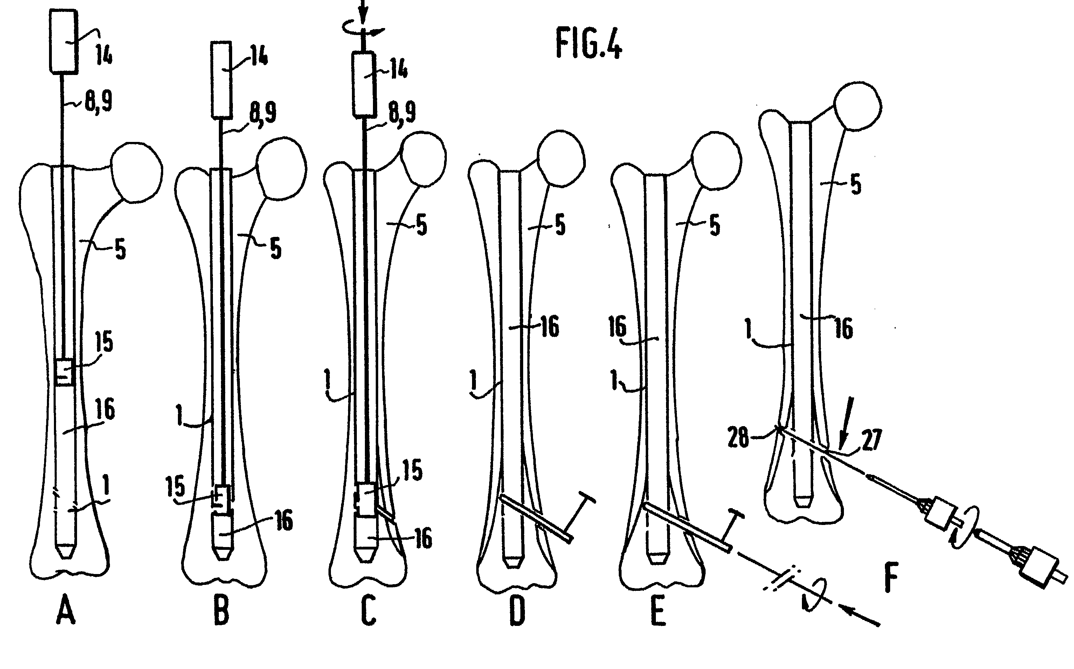

Les schémas A à F de la figure 4 représentent différentes étapes successives de l'utilisation de l'équipement selon l'invention pour la consolidation d'un fémur.

Après la mise en place du clou 1 dans la cavité médullaire du fémur 5, on introduit, à l'aide de la tige 23, le bloc de guidage 15 dans la cavité 16 du clou 1 (figure 4 A). Lorsque l'engagement de la bille 20 dans le trou de positionnement 21 du clou indique que le bloc 15 a atteint la position voulue, dans laquelle l'orifice 22 du passage 17 est en registre avec le trou de vis de sortie 2 du clou (figure 4 B), on fait tourner l'arbre flexible 8 à l'aide du moteur 14 et l'on agit sur cet arbre de manière à faire avancer la mèche 12 à travers le trou de vis 2 et à percer dans la corticale adjacente un trou qui sera donc parfaitement aligné sur le trou de vis 2.

On met en place ensuite, d'une manière connue en soi, un guide de perçage par voie extérieure (figure 4 C), ce qui permet la mise en oeuvre d'une broche de Kirchner traversant le clou et les deux régions corticales de part et d'autre de celui-ci (figures 4 D et E).

En s'aidant de cette broche, servant de tige-guide, on procède alors à une opération d'alésage, en 27, de la corticale adjacente au trou de vis de sortie 3 à un diamètre intérieur égal au diamètre du fond de filet de la vis 4 (figure 5) à mettre en place, cependant que l'autre corticale est alésée en 28 au diamètre du haut de filet de cette vis, ces deux opérations successives d'alésage étant effectuées à l'aide de deux forets indiqués schématiquement, sur la figure 4 F.

La figure 5 montre le fémur 5 muni du clou intramédullaire 1, après l'alésage des corticales en 27 et 28, ainsi que la vis 4, avant la mise en place de celle-ci, et la figure 6 est une vue analogue à la figure 5, montrant, dans sa partie inférieure, le fémur 5, le clou 1 et la vis 4 mise en place définitivement.

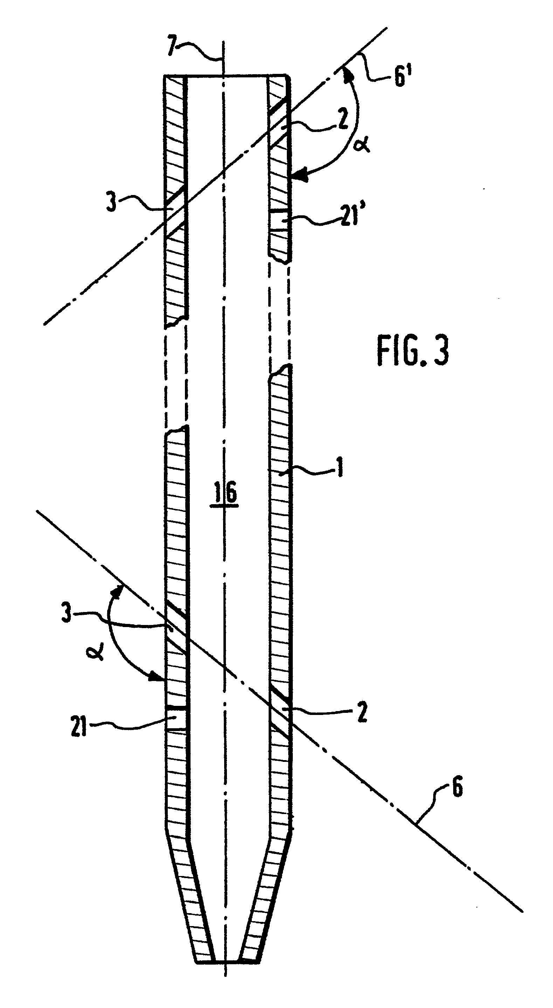

En introduisant le bloc de guidage 15 dans la cavité 16 du clou dans une position décalée de 180° par rapport à celle considérée dans la description qui précède, on peut, ainsi que l'indique schématiquement la partie supérieure de la figure 3, percer l'os à l'aide du flexible 8 et de la mèche 12 sur le côté opposé à celui considéré ci-dessus, à condition, bien entendu, de décaler également de 180° la disposition des trous de vis et du trou de positionnement. Ceci est symbolisé par l'emploi, sur la partie supérieure de la figure 3, des mêmes références numériques que celles utilisées sur les figures précédentes et sur la partie inférieure de la figure 3, mais pourvues du signe "prime" ('). On comprendra que, dans ces conditions, l'axe commun 6' est également incliné sous un même angle alpha par rapport à l'axe médian 7 du clou, mais en sens inverse par comparaison à la disposition décrit ci-dessus.

On peut ainsi, en cas de besoin, fixer un même clou intramédullaire 1 à l'aide de deux vis 4 et 4' logées respectivement dans des trous 27, 28 et 27', 28' et inclinées sous des angles égaux (alpha), mais dans des sens opposés, ainsi que le montre la figure 6.

Selon le mode de réalisation qui vient d'être décrit, le guidage de l'arbre flexible 8 est assuré d'une façon permettant sa rotation à une vitesse relativement élevée, mais le perçage en biais dans la corticale de l'os se révèle difficile à réaliser car la tête de perçage tend à être déviée par l'attaque en biais par la mèche 12 de la surface intérieure de la paroi dure de l'os.

Selon un autre mode de réalisation de la sonde de perçage et du clou intramédullaire permettant d'éliminer ces derniers inconvénients tout en évitant d'avoir recours à des procédures fastidieuses et dangereuses telles que la radioscopie de l'os au cours des forages, la gaine présente, au niveau du bloc de guidage, une paroi rigide qui vient déboucher en face de la paroi intérieure du clou dans une position sensiblement perpendiculaire à cet paroi. La gaine présente, sur toute sa longueur, une paroi rigide à l'intérieur de laquelle se déplace axialement l'arbre flexible pour contrôler l'avance et le recul de la tête de perçage.

L'arbre flexible est constitué d'une gaine flexible, rigide ou quasi rigide en compression, par exemple une gaine métallique, mobile axialement et à l'intérieur de laquelle est montée une tige flexible rotative présentant une grande résistance aux sollicitations en flexion-rotation, par exemple en racine de nylon, et portant respectivement, à son extrémité distale, une tête de perçage destinée à forer la corticale de l'os à partir du passage de perçage ménagé dans la paroi du clou et, à son extrémité proximale, une tête d'entraînement en rotation.

Selon un autre mode de réalisation de la sonde de perçage, la tête de perçage présente une surface d'appui permettant à la gaine flexible de pousser la tête de perçage pour provoquer son avance au cours de son entraînement en rotation par la tige flexible, sans exercer d'effort axial sur cette tige flexible. La tête de perçage et/ou la tête d'entraînement en rotation sont, de préférence, rapportées par sertissage sur la tige flexible monobloc.

Le clou intramédullaire, comportant une cavité interne apte à recevoir une sonde de perçage selon l'invention et présentant un profil transversal sensiblement constant et de forme générale tubulaire et des trous de vis ou de broches d'entrée et de sortie alignés, son extrémité distale étant légèrement en pointe tandis que son extrémité proximale est munie d'au moins un passage de vis incliné par rapport à l'axe médian longitudinal dudit clou, est caractérisé selon ce dernier mode de réalisation de l'invention en ce qu'il comporte à son extrémité distale, avant la zone en pointe, d'une part au moins deux couples de trous ou passages alignés transversalement à l'axe du clou pour le passage de vis de fixation de l'os traversant le clou et, d'autre part, des moyens de positionnement et de verrouillage de la sonde de perçage, correspondant chacun à l'un des couples de trou.

Selon un mode de réalisation avantageux, les moyens de positionnement sont constitués par un trou spécifique différent de ceux des couples de trous et destiné à recevoir une bille ou une tige en saillie portée par la sonde de guidage.

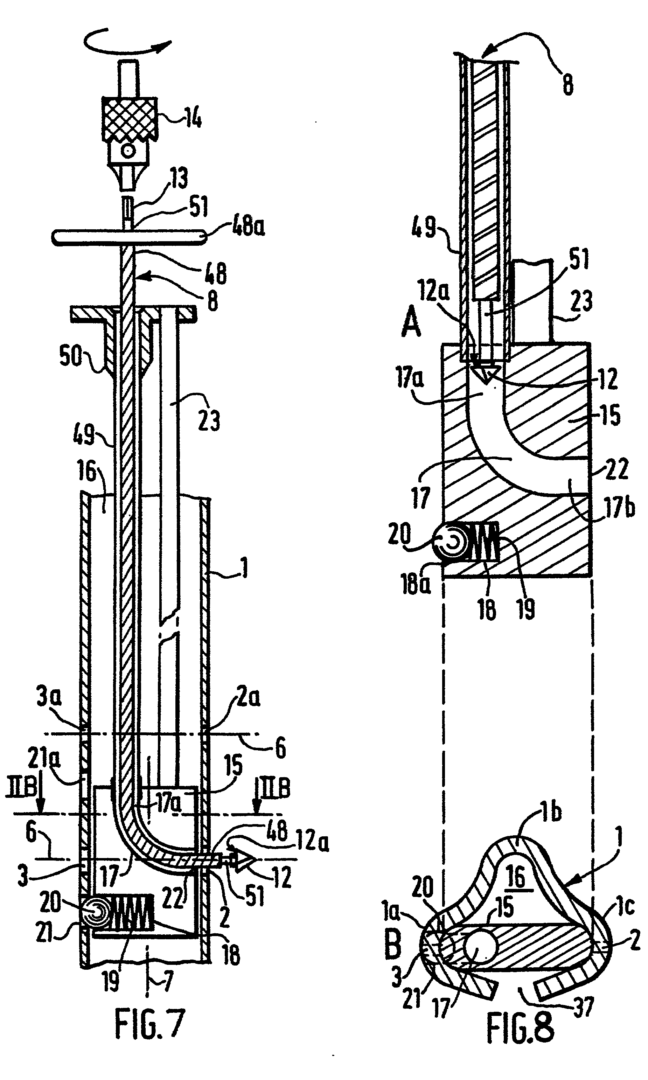

Dans le mode de réalisation selon les figures 7 à 9, où les éléments identiques à ceux des figures 1 à 6 sont dotés des mêmes numéros de référence, la sonde selon l'invention comprend un arbre flexible 8 monté à rotation dans une gaine tubulaire rigide 49 pourvue, à l'une de ses extrémités (appelée ci-après extrémité proximale) d'une poignée tubulaire 50 solidaire de la gaine 49 et apte à faciliter l'introduction de l'arbre flexible dans celle-ci ainsi que son retrait.

L'arbre flexible 8 est constitué d'une gaine flexible 48, rigide ou quasi rigide en compression, à l'intérieur de laquelle est montée à rotation une tige flexible 51 très résistante à la flexion et constituée, par exemple, d'un fil épais ou racine ou âme en nylon et qui porte, à l'une de ses extrémités (appelée ci-après extrémité distale), un outil de forage ou de perçage tel qu'une mèche 12 du type couramment utilisé en chirurgie pour le perçage des os. A son autre extrémité, ou extrémité proximale, la tige flexible 51 est munie de moyens d'accouplement 13, par exemple un manchon de section carrée, à un moteur rotatif 14. La mèche 12 et le manchon 13 formant tête d'entraînement en rotation sont, de préférence, sertis sur la tige flexible 51.

La gaine 49 est fixée, par son extrémité opposée à la poignée 50 ou extrémité distale, sur un bloc de guidage 15 présentant un contour tel qu'il puisse être introduit dans la cavité 16 définie à l'intérieur du clou 1 et qu'il puisse y être déplacé à coulissement dans la direction de l'axe 7 du clou tout en étant immobilisé en rotation par rapport à celui-ci. A cet effet, le bloc 15 épouse, dans le présent exemple, le profil intérieur concave des deux lobes opposés 1a et 1c du clou 1 ainsi qu'il ressort, notamment, de la coupe transversale représentée en B sur la figure 8.

Le bloc de guidage 15 est pourvu d'un passage interne 17 dont la section transversale correspond à celle de l'alésage ou passage défini à l'intérieur de la gaine 49 pour prolonger cette gaine rigide et servir de logement à l'arbre flexible 8. Une première partie ou partie d'entrée 17a du passage 17 est alignée sur la partie terminale distale de la gaine 49 et s'étend donc, de façon sensiblement parallèle à l'axe médian longitudinal 7 du clou 1, lorsque le bloc 15 est placé dans la cavité 16 du clou, cependant qu'une second partie ou partie distale 17b dudit passage qui aboutit à un orifice de sortie 22, forme avec la partie proximale 17a un arrondi sensiblement en quart de cercle qui amène l'orifice 22 à déboucher de façon sensiblement perpendiculaire à la paroi du clou 1. Le rayon de l'arrondi du passage 17 est choisi le plus grand possible dans les limites dimensionnelles imposées par le bloc de guidage 15, de manière à limiter la fatigue de la gaine flexible 48 et de la tige flexible 51 et à faciliter la transmission du couple de rotation à cette tige 51 et l'action de poussée exercée sur la gaine flexible 48. Le passage 17 peut bien entendu être remplacé par un prolongement de la gaine rigide 49 débouchant radialement en 22, la gaine 49 étant fixée rigidement au bloc de guidage 15 qu'il n'est alors plus nécessaire de traverser par un passage 17 en arc de cercle. La gaine flexible 48 peut être immobilisée en rotation ou bien être laissée libre de tourner sous l'effet d'entrainement de la tige flexible 51. On peut aussi entrainer en rotation la gaine flexible 48, soit en synchronisme avec la tige flexible 51, soit à une vitesse différente, soit en sens inverse, par exemple pour rigidifier l'ensemble de la gaine flexible 48 et de la tige 51 à la sortie de l'orifice 22.

Le bloc de guidage 15 est muni de moyens de verrouillage en positions dans le clou 1. Ces moyens de verrouillage sont constitués encore, dans ce mode de réalisation, d'un logement cylindrique borgne 18 s'étendant perpendiculairement à l'axe 7 (en référence au bloc 15 introduit dans le clou 1) et renfermant un ressort hélicoïdal 19 coaxial audit logement et qui sollicite, vers l'extérieur du bloc 15, une bille de positionnement 20 retenue en butée dans le logement 18 par un rebord 18a mais venant en saillie sous l'action du ressort 19 hors de l'orifice de sortie du logement. La bille 20 coopère par encliquetage avec une cavité de positionnement formée dans le présent exemple par un trou 21 ménagé dans la paroi du clou 1 dans le lobe opposé à celui qui présente les trous de vis d'entrée 2. L'emplacement du trou de positionnement 21 est choisi de façon telle, compte tenu de l'agencement du bloc de guidage, que la bille 20 pénètre partiellement dans le trou 21 lorsque l'orifice de sortie 22 du passage 17 se trouve aligné sur le trou de vis d'entrée 2 du clou 1. Dans le mode de réalisation représenté, le trou de positionnement 21 et le trou de vis de sortie 3 ont leurs centres respectifs placés sur une même génératrice de la paroi du clou 1. Afin d'éviter des erreurs de positionnement, le trou de positionnement 21 présente également un diamètre sensiblement plus grand que celui du trou de vis de sortie 3 et l'on utilise une bille 20 de diamètre relativement grand qui ne risque pas, lors de la mise en place du bloc 15, de produire un effet d'encliquetage dans le trou de vis 3, ce qui pourrait être interprété à tort comme indiquant que le bloc 15 a atteint sa position de verrouillage dans la cavité 16 du clou.

Afin de faciliter sa manipulation et son positionnement, le bloc 15 est muni d'un autre tube rigide 23 s'étendant dans la même direction générale que la gaine 49 et qui peut être utilisée en plus à des fins d'observation ou de commande du verrouillage de bloc 15.

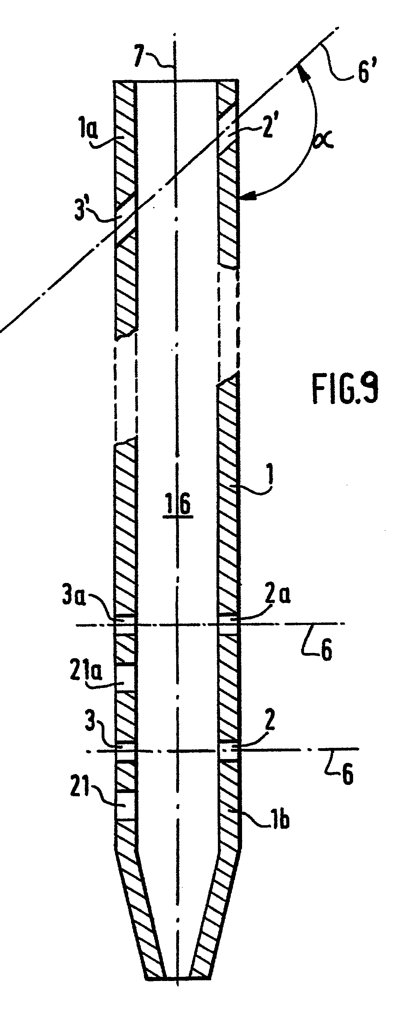

On voit, sur la figure 9, un clou intramédullaire 1 représenté en coupe et dont la partie centrale est supprimée pour réduire l'encombrement du dessin. Dans sa partie proximale la destinée à être montée à proximité de la tête d'un fémur, le clou 1 porte deux trous 2' et 3' alignés selon un axe 6' incliné d'un angle alpha par rapport à l'axe longitudinal 7 du clou 1. De façon classique, le chirurgien que bénéficie d'une bonne vision sur la partie proximale, peut forer en biais dans la tête du fémur un alésage en correspondance avec les trous 2' et 3' pour placer dans la corticale de l'os une vis de fixation qui traverse les trous 2' et 3'.

La partie distale 1b du clou 1 présente, par contre, au moins deux séries de trous alignés pour des vis de fixation traversant la corticale de l'os selon une direction sensiblement perpendiculaire à l'axe 7 du clou 1 et donc à l'axe longitudinal de l'os (par exemple un fémur) dans la partie médullaire duquel est implanté le clou 1. Les trous alignés sont repérés 2, 3 et 2a, 3a sur la figure 3 et à chaque couple de trous correspond un trou de plus grand diamètre 21 et 21a destiné à recevoir la bille 20 pour le verrouillage en position du bloc 15.

La mise en place des vis de fixation distales dans les trous alignés 2, 3 et 2a, 3a s'effectue de la façon suivante. Après la mise en place du clou 1 dans la cavité médullaire d'un os tel qu'un fémur, on introduit en s'aidant du tube 23, le bloc de guidage 15 dans la cavité 16 du clou 1. Lorsque l'engagement de la bille 20 dans le trou de positionnement 21 du clou indique que le bloc 15 a atteint la position de forage, dans laquelle l'orifice 22 du passage 17 vient se placer en face du trou de vis de sortie 2 du clou, on fait tourner la tige flexible 51 à l'aide du moteur 14 et l'on repousse la gaine 48 vers le bas à l'aide d'un plateau 48a solidaire de cette gaine 48, de manière à plaquer l'autre extrémité de cette gaine 48 au contact d'une surface d'appui 12a ménagée à l'arrière de la mèche 12. Sous l'effet de la poussée de la gaine 48 flexible mais quasi rigide en compression, la mèche 12 qui tourne vient s'appliquer sur la corticale adjacente de l'os et forer perpendiculairement à la paroi interne de cet os, un trou ou alésage qui sera parfaitement aligné sur le trou de vis 2.

On met ensuite en place, d'une manière connue en soi, un guide de perçage par voie extérieure et le chirurgien réalise un perçage transversal traversant l'os et les trous 2 et 3, ce qui permet la mise en oeuvre d'une broche de Kirchner traversant le clou et les deux régions corticales de l'os de part et d'autre de celui-ci et, le cas échéant, le remplacement de la broche de Kirchner par une vis de fixation du clou 1 sur la corticale de l'os. On procède de la même façon pour les trous suivants repérés 2a et 3a en déplaçant le bloc 15 jusqu'au verrouillage de la bille 20 dans le trou de plus grand diamètre 21a.

**Revendications**

1.- Sonde destinée à venir se loger à l'intérieur d'un clou tubulaire intramédullaire à partir d'une extrémité accessible du clou pour forer un passage ou un alésage dans la paroi corticale d'un os qui porte le clou, caractérisé en ce qu'elle comporte un bloc de guidage et de positionnement (15) qui est guidé et mobile axialement dans la cavité interne (16) du clou (1) et qui supporte une gaine (9, 17, 49) traversée intérieurement par un arbre flexible (8) portant à son extrémité distale une tête de perçage (12) et à son extrémité proximale des moyens d'accouplement (13) à un moteur rotatif (14) et en ce que ladite gaine (9, 19, 49) débouche à son extrémité proximale axialement à partir de la cavité interne (16) du clou et respectivement à son extrémité distale, latéralement en direction de la paroi intérieure du clou par un orifice distal (22) situé en face d'un trou de vis (2) ménagé dans la paroi du clou (1), ledit bloc (15) comportant en outre des moyens de positionnement (18, 19, 20) aptes à coopérer avec des moyens de positionnement complémentaires (21) du clou intramédullaire (1), de manière à positionner la sortie proximale de la gaine (9, 17, 49) en face du trou de vis (2) correspondant.

2.- Sonde selon la revendication 1, caractérisée en ce que lesdits moyens de positionnement du bloc de guidage comportent une bille (20) montée de manière mobile dans un logement (18) ménagé dans ledit bloc (15) selon un axe sensiblement transversal à la direction de coulissement dudit bloc dans le clou (1) et décalé angulairement par rapport à l'orifice (22) de sortie distale de la gaine (2, 17, 49), cette bille (20) étant sollicitée par un ressort (19) pour faire saillie à la surface extérieure dudit bloc de manière à pouvoir coopérer avec lesdits moyens de positionnement complémentaires du clou (1) qui sont constitués par une cavité de positionnement (21) ménagée dans la paroi du clou dans une position déterminée par rapport audit trou de vis (2) du clou, de manière que l'engagement de la bille (20) dans la cavité de positionnement (21) se produise lorsque l'orifice distal (22) de la gaine (9, 17, 49) est sensiblement aligné sur le trou de vis (2) du clou.

3.- Sonde de guidage selon la revendication 2, caractérisée en ce que la cavité de positionnement est constituée par un trou ou passage de positionnement (21) traversant la paroi du clou (1).

4.- Sonde de guidage selon la revendication 2 ou 3, caractérisée en ce que le logement (18) ainsi que l'orifice distal (22) de la gaine (9, 17, 49) débouchent dans des zones du bloc de guidage (15) qui sont en contact direct avec la paroi interne du clou (1) lorsque ledit bloc (15) est introduit dans ce dernier.

5.- Sonde de guidage selon une quelconque des revendications 1 à 4, caractérisée en ce que le bloc de guidage (15) est muni d'une tige ou tube axial de manipulation (23) s'étendant dans la même direction générale que la partie proximale de la gaine (9, 17, 49) et comporte une surface de préhension (24, 10, 50) qui dépasse de l'extrémité proximale dudit clou (1) pour toutes les positions longitudinales dudit bloc de guidage (15) à l'intérieur du clou.

6.- Sonde de perçage selon l'une des revendications 1 à 5, caractérisée en ce que le bloc de guidage et de positionnement (15) est muni d'un passage interne (17) qui est raccordé à l'alésage de la gaine (9) et présente une section transversale identique à celle de cet alésage, ledit passage (17) comportant une première partie de passage (17a) alignée sur l'alésage de la gaine (9) et une seconde partie de passage (17b) formant avec ladite première partie (17a) un angle obtus ($\alpha$) et débouchant à la surface dudit bloc de guidage (15) par un orifice (22) susceptible d'être aligné sur un trou de vis d'entrée (2) ménagé dans la paroi du clou intramédullaire (1).

7.- Sonde selon l'une des revendications 1 à 5, caractérisée en ce que la gaine (49, 17) présente, au niveau du bloc du guidage (15), une paroi rigide qui vient déboucher en face de la paroi intérieure du clou (1) dans une position sensiblement perpendiculaire à cette paroi.

8.- Sonde selon la revendication 7, caractérisée en ce que la gaine (49, 17) présente, sur toute sa longueur, une paroi rigide à l'intérieur de laquelle se déplace axialement l'arbre flexible (8) pour contrôler l'avance et le recul de la tête de perçage (12).

9.- Sonde selon la revendication 7 ou 8, caractérisée en ce que l'arbre flexible (8) est constitué d'une gaine flexible (48) rigide ou quasi rigide en compression, mobile axialement et à l'intérieur de laquelle est montée une tige flexible rotative (51) présentant une grande résistance aux sollicitations en flexion-rotation, et portant respectivement à son extrémité distale, une tête de perçage (12) destinée à forer la corticale de l'os à partir du passage de perçage (2, 3) ménagé dans la paroi du clou (1) et, à son extrémité proximale, une tête (13) d'entraînement en rotation.

10.- Sonde selon la revendication 9, caractérisée en ce que la tête de perçage (12) présente une surface d'appui (12a) permettant à la gaine flexible (48) de pousser la tête de perçage pour provoquer son avance au cours de son entraînement en rotation par la tige flexible (51), sans exercer d'effort axial sur cette tige flexible.

11.- Sonde selon la revendication 9 ou 10, caractérisée en ce que la tête de perçage (12) et/ou la tête d'entraînement en rotation (13) est (sont) rapportées par sertissage sur la tige flexible (51).

12.- Clou intramédullaire comportant une cavité interne apte à recevoir une sonde de perçage selon l'une quelconque des revendications 7 à 11 et présentant un profil transversal sensiblement constant et de forme générale tubulaire et des trous de vis ou de broches d'entrée et de sortie alignés, son extrémité distale étant légèrement en pointe tandis que son extrémité proximale est munie d'au moins un passage de vis incliné par rapport à l'axe médian longitudinal dudit clou, caraotérisé en ce qu'il comporte à son extrémité distale (1b), avant la zone en pointe, d'une part au moins deux couples de trous ou passages (2, 3; 2a, 3a) alignés transversalement à l'axe (7) du clou (1) pour le passage de vis de fixation de l'os traversant le clou et d'autre part, des moyens de positionnement et de verrouillage (21, 21a) ménagés dans la paroi du clou et appelés à coopérer avec des moyens correspondants de la sonde de perçage, lesdits moyens de positionnement correspondant chacun à l'un des couples de trous.

13.- Clou selon la revendication 12, caractérisé en ce que les moyens de positionnement sont constitués par un trou (21, 21a) spécifique différent de ceux des couples de trous (2, 3; 2a, 3a) et destiné à recevoir une bille (20) ou une tige en saillie portée par la sonde de guidage.

14.- Clou intramédullaire creux propre à être utilisé en combinaison avec la sonde de perçage selon la revendication 6, présentant un profil transversal non circulaire, constant sur la majeure partie de sa longueur et pourvu, au moins dans la partie distale de sa paroi, d'au moins un trou de vis d'entrée et d'au moins un trou de vis de sortie aligné selon un même axe commun sur le trou de vis d'entrée, caractérisé en ce que l'axe commun d'alignement (6) des trous de vis d'entrée (2) et de sortie (3) est incliné par rapport à l'axe médian longitudinal (7) dudit clou (1) sous un angle (α) égal à l'angle d'inclinaison de la seconde partie (17b) du passage (17) dudit bloc de guidage (15) de ladite sonde de perçage par rapport à la première partie (17a) de ce passage, et en ce qu'une cavité de positionnement (21) appelée à coopérer avec la bille de positionne ment (20) dudit bloc (15) est ménagée dans la paroi du clou, dans une partie de celle-ci qui est appelée à entrer en contact direct avec la surface dudit bloc (15) lorsque celui-ci est amené, par coulissement dans le clou, dans la zone de cette partie de paroi.

15.- Clou intramédullaire selon la revendication 14, caractérisé en ce que ladite cavité de positionnement est constituée par un trou de positionnement (21) traversant la paroi du clou (1).

16.- Clou intramédullaire selon la revendication 15, caractérisé en ce que l'axe du trou de positionnement (21) est situé dans le même plan que l'axe commun (6) desdits trous de vis d'entrée et de sortie (2, 3).

17.- Clou intramédullaire selon la revendication 15 ou 16, caractérisé en ce que le diamètre du trou de positionnement (21) est sensiblement plus grand que celui du trou de vis de sortie (3).

18.- Clou intramédullaire selon l'une des revendications 15 à 17, caractérisé en ce que l'axe du trou de positionnement (21) est situé dans un plan décalé angulairement par rapport au plan contenant l'axe commun (6) des trous de vis d'entrée et de sortie (2, 3).

19.- Clou intramédullaire selon une quelconque des revendications 14 à 18, caractérisé en ce que sa section transversale présente une pluralité de lobes (1a, 1b, 1c), le profil extérieur de bloc de guidage (15) épousant au moins un desdits lobes.

20.- Clou intramédullaire selon la revendication 19, caractérisé en ce que lesdits lobes (1a, 1b, 1c) sont au nombre de trois, le profil extérieur dudit bloc de guidage (15) épousant deux de ces lobes (1a, 1c).

**Claims**

1. Boring sensor intended to be housed within an intramedullary tubular nail from an accessible end of the nail in order to drill a passage or a bore in the cortical wall of a bone that bears the nail, characterized in that it comprises a guiding and positioning unit (15) which is guided and axially movable in the internal recess (16) of the nail (1) and which bears a sheath 9, 17, 49) through which passes a flexible shaft (8) bearing at its distal end a boring head (12) and at its proximal end means (13) for coupling to a rotary motor (14) and in that said sheath (9, 19, 49) issues at its proximal end axially from the internal recess (16) of the nail and respectively at its distal end, laterally in the direction of the internal wall of the nail by a distal orifice (22) facing a screw hole (2) provided in the wall of the nail (1), the said unit (15) furthermore comprising positioning means (18, 19, 20) adapted to cooperate with the complementary positioning means (21) of the intramedullary nail (1), so as to set in position the proximal outlet of the sheath (9, 17, 49) facing the corresponding screw hole.

2. Sensor according to claim 1, characterized in that the said positioning means for the guiding unit comprise a ball (20) mounted so as to be movable within a housing (18) provided within said unit (15) according to an axis substantially transversal to the sliding direction of the said unit in the nail (1) and angularly shifted with respect to the distal outlet orifice or opening (22) of the sheath (2, 17, 49), this ball (20) being urged by a spring (19), in order to protrude from the external surface of the said unit in such a way as to be able to cooperate with the said complementary positioning means of the nail (1) that are constituted by a positioning recess (21) provided in the wall of the nail in a position determined with respect to the screw hole (2) of the nail, so that the engagement of the ball (20) within the positioning recess (21) is produced when the distal orifice (22) of the sheath (9, 17, 49) is substantially aligned upon the screw hole (2) of the nail.

3. Guiding sensor according to claim 2, characterized in that the said positioning recess is constituted by a positioning hole or passage (21) crossing through the wall of the nail (1).

4. Guiding sensor according to claim 2, characterized in that the housing (18) as well as the distal orifice (22) of the sheath (9, 17, 49) issue into zones of the guiding unit (15) that are in direct contact with the internal wall of the nail (1) when the said unit (15) is introduced into said nail.

5. Guiding sensor according to any one of claims 1 to 4, characterized in that the guiding unit (15) is provided with an axial handling rod or tube (23) extending in the same general direction as the proximal part of the sheath (9, 17, 49) and comprises a gripping surface (24, 10, 50) that projects out of the proximal end of the said nail (1) for any given longitudinal position of the said guiding unit (15) within the nail.

6. Boring sensor according to any one of claims 1 to 5, characterized in that the guiding and positioning unit (15) is provided with an internal passage (17) that is associated to the bore of the sheath (9) and presents a cross-section identical to that of said bore, the said passage (17) comprising a first portion (17a) aligned upon the bore of the sheath (9) and a second portion (17b) forming, with the said first portion (17a), an obtuse angle (α) and issuing towards the surface of the said guiding unit (15) through an orifice or opening (22) adapted to be aligned upon an inlet screw hole (2) provided in the wall of the intramedullary nail (1).

7. Claim according to any of claims 1 to 5, characterized in that the sheath (49, 17) presents, in the guiding unit (15), a rigid wall and issues opposite the internal wall of the nail (1) in a position substantially perpendicular to this wall.

8. Sensor according to claim 7, characterized in that the sheath (49, 17) presents, along its whole length a rigid wall within which is axially moved the flexible shaft (8) in order to control the feed and withdrawal movements of the boring head (12).

9. Sensor according to claim 7 or 8, characterized in that the flexible shaft (8) comprises a flexible sheath (48), rigid or quasi rigid in compression, axially movable and inside of which is mounted a rotary flexible rod (51) presenting a high resistance to flexion-rotation stresses, and respectively bearing at its distal end, a boring head (12) intended to drill the cortical of the bone from the drilling passage (2, 3), provided in the wall of the nail (1), and at its proximal end, a driving in rotation head (13).

10. Sensor according to claim 9, characterized in that the boring head (12) presents a bearing surface (12a) allowing the flexible sheath (48) to push the boring head in order to provoke its moving forward during its driving in rotation by the flexible rod (51), without exerting any axial effort upon this flexible rod.

11. Sensor according to claim 9 or 10, characterized in that the boring head (12) and/or the driving in rotation head (13) is (are) secured onto the flexible rod (51) by crimping.

12. Intramedullary nail with an internal cavity adapted to receive a boring sensor according to any one of claims 7 to 11 and presenting a substantially constant transversal outline having a generally tubular form and aligned inlet and outlet screw or pin holes, its distal end being slightly pointed while its proximal end is provided with at least one screw passage inclined with respect to the longitudinal median axis of the said nail, wherein it comprises at its distal end (1b), prior to the pointed zone, on the one hand at least two pairs of holes or passages (2, 3; 2a, 3a) transversally aligned with respect to the axis (7) of the nail (1) for the passage of fixation screws of the bone crossing through the nail and on the other hand, positioning and locking means (21, 21a) provided in the wall of the nail and adapted to cooperate with corresponding means, the said positioning means of the boring sensor, the positioning means each corresponding to one of the pairs of holes.

13. Nail according to claim 12, characterized in that the positioning means are constituted by a specific hole (21, 21a) different from those of the pairs of holes (2, 3; 2a, 3a) and intended to receive a protruding ball (20) or rod borne by the guiding sensor.

14. Intramedullary nail with an internal cavity that is adapted to receive the boring sensor according to claim 6, presenting a non-circular transversal outline, constant on the major portion of its length and provided, at least in the distal portion of its wall, with at least one inlet screw hole and at least one outlet screw hole aligned according to a common axis upon the inlet screw hole wherein the common axis of alignment (6) of the inlet (2) and outlet (3), screw holes is inclined with respect to the longitudinal median axis (7) of the said nail (1) under an angle alpha equal to the slope angle of the second portion (17b), of the passage (17) of the said guiding unit (15) of said boring sensor with respect to the first portion (17a), of this passage and in that a positioning recess (21) adapted to cooperate with the positioning ball (20) of the said unit (15) is provided in the wall of the nail in a part of the latter that is adapted to enter into direct contact with the surface of the said unit (15) when said unit is brought, through sliding in the nail, into the zone of this portion of the wall.

15. Intramedullary nail according to claim 14, characterized in that the said positioning recess is constituted by a positioning hole (21) crossing through the wall of the nail (1).

16. Intramedullary nail according to claim 15, characterized in that the axis of the positioning hole (21) is situated in the same plane as the common axis (6) of the said inlet and outlet screw holes (2, 3).

17. Intramedullary nail according to claim 15 or 16, characterized in that the diameter of the positioning hole (21) is substantially larger than that of the outlet screw hole (3).

18. Intramedullary nail according to one of claims 15 to 17, wherein the axis of the positioning hole (21) is situated in a plane that is angularly shifted with respect to the plane containing the common axis (6) of the inlet and outlet screw holes (2, 3).

19. Intramedullary nail according to any one of claims 14 to 18, characterized in that its transversal cross-section presents a plurality of lobes (1a, 1b, 1c) the external outline of the guiding unit (15) matching at least one of the said lobes.

20. Intramedullary nail according to claim 19, characterized in that there are three such lobes (1a, 1b, 1c) the external outline of the guiding unit (15) matching two of these lobes (1a, 1c).

**Patentansprüche**

1. Sonde zur Einführung in einen hohlen Knochennagel von dessen zugänglichem Ende aus, um in der Seitenwand des den Knochennagel aufnehmenden Knochens eine Bohrung zu erzeugen, dadurch gekennzeichnet, dass sie einen Führungsblock (15) aufweist, der im Innenraum (16) des Knochennagels (1) axial beweglich und geführt ist, der eine Hülle (9, 17, 49) trägt, in der eine flexible Drehwelle (8) angeordnet ist, die an ihrem Distalende einen Bohrkopf (12) und an ihrem Proximalende Kupplungsmittel (13) für einen Drehmotor (14) aufweist, und das die Hülle (9, 17, 49) an ihrem Proximalende axial von dem Innenraum (16) des Knochennagels mündet und an ihrem Distalende quer zur Innenwand des Knochennagels durch eine Austrittsöffnung (22) mündet, die gegenüber einem Eintrittsloch (2) in der Wandung des Knochennagels liegt, wobei der Führungsblock (15) ferner Positionierungsmittel (18, 19, 20) aufweist, die mit komplementären Positionierungsmitteln (21) des Knochennagels (1) zusammenwirken, um die Austrittsöffnung (22) der Hülle (9, 17, 49) gegenüber dem entsprechenden Schraubenloch (2) zu positionieren.

2. Sonde nach Anspruch 1, dadurch gekennzeichnet, dass die Positionierungsmittel des Führungsblocks eine Positionierungskugel (20) aufweisen, die beweglich angeordnet ist in einer im Führungsblock (15) vorgesehenen Sackbohrung (18), welche sich quer zur Richtung der Verschiebung des Führungsblocks (15) in dem Knochennagel (1) erstreckt und in bezug auf die Austrittsöffnung (22) der Hülle (2, 17, 49) winkelversetzt ist, wobei diese Kugel (20) von einer Feder (19) derart beaufschlagt wird, dass sie die Aussenfläche des Führungsblocks (15) überragt und mit den komplementären Positionierungsmitteln des Knochennagels (1) zusammenwirkt wobei die komplementären Positionierungsmittel als Positionierungsbohrung (21) gestaltet sind, die in der Wand des Knochennagels in einer bestimmten Lage in bezug auf das Eingangsloch (2) des Knochennagels derart angeordnet ist, dass die Kugel (20) in die Positionierungsbohrung (21) dann einrastet, wenn die Austrittsöffnung (22) der Hülle (9, 17, 49) sich im wesentlichen mit dem Schraubloch (2) des Knochennagels deckt.

3. Sonde nach Anspruch 2, dadurch gekennzeichnet, dass die Positionierungsbohrung (21) eine die Wandung des Knochennagels 1 durchquerende Positionierungs-Durchgangsbohrung (21) ist.

4. Sonde nach Anspruch 2 oder 3, dadurch gekennzeichnet, dass die Sackbohrung (18) und die Austrittsöffnung (22) der Hülle (9, 17, 49) in Bereichen des Führungsblocks (15) münden, die mit der Innenwandung des Knochennagels (1) in unmittelbarer Berührung stehen, wenn der Block (15) in den Knochennagel eingesetzt ist.

5. Sonde nach einem der Ansprüche 1 bis 4, dadurch gekennzeichnet, dass der Führungsblock (15) eine axiale Handhabungsstange (23) besitzt, die sich im wesentlichen in der gleichen Richtung erstreckt wie der Proximalteil der Hülle (9, 17, 49) und einen Handgriff (24, 10, 50) aufweist, der das Proximalende des Knochennagels (1) bei jeder Längslage des Führungsblocks (15) im Innenraum des Knochennagels überragt.

6. Sonde nach einem der Ansprüche 1 bis 5, dadurch gekennzeichnet, dass der Führungsblock (15) einen Kanal (17) besitzt, der an der Hülle (9) angeschlossen ist und einen dem Querschnitt des Hülleninnenraums gleichen Querschnitt besitzt und ferner einen Proximalteil (17a) aufweist, welcher auf den Hülleninnenraum ausgerichtet ist, sowie einen Distalteil (17b), der mit dem Proximalteil (17a) einen stumpfen Winkel Alpha einschliesst und an der Oberfläche des Führungsblocks (15) in einer Öffnung (22) mündet, die auf ein in der Wandung des Knochennagels (1) vorgesehenes Eingangsloch (2) ausrichtbar ist.

7. Sonde nach einem der Ansprüche 1 bis 5, dadurch gekennzeichnet, dass die Hülle (49, 17) im Bereich des Führungsblocks (15) eine steife Wandung aufweist, die im wesentlichen senkrecht zur Innenwand des Knochennagels mündet.

8. Sonde nach Anspruch 7, dadurch gekennzeichnet, dass die Hülle (49, 17) über ihre gesamte Länge eine steife Wandung aufweist, innerhalb welcher die biegsame Welle (8) axial verschiebbar ist, um die Vorwärts- und Rückwärtsbewegung des Bohrkopfs (12) zu bewirken.

9. Sonde nach Anspruch 7 oder 8, dadurch gekennzeichnet, dass die biegsame Welle (8) aus einer flexiblen, im wesentlichen druckfesten Hülle besteht, die axial beweglich ist und innerhalb welcher eine gegen Biege- und Drehbeanspruchung widerstandsfähige flexible Stange (51) drehbar angeordnet ist und welche an ihrem Distalende einen Bohrkopf (12) zum Durchbohren der Knochenwandung, ausgehend von dem Eingangs- und Ausgangsloch (2, 3) des Knochennagels (1), an ihrem Proximalende hingegen eine Kupplung (13) trägt.

10. Sonde nach Anspruch 9, dadurch gekennzeichnet, dass der Bohrkopf (12) eine Andrückfläche (12a) aufweist, vermittels welcher die flexible Hülle (48) den Bohrkopf (12) nach vorne drücken kann, um während seiner auf ihn durch die flexible Stange (51) übertragenen Drehbewegung seinen Vorschub zu bewirken, ohne dass hierbei axiale Kräfte auf die flexible Stange (51) einwirken.

11. Sonde nach Anspruch 9 oder 10, dadurch gekennzeichnet, dass der Bohrkopf (12) und/oder der Drehantriebskopf (13) mit der flexiblen Stange versickt ist (sind).

12. Knochennagel mit einem Innenraum 16, in den eine Sonde nach einem der Ansprüche 7 bis 11 einführbar ist, und ein im wesentlichen konstantes Querprofil aufweist, wobei der Knochennagel im wesentlichen rohrförmig gestaltet ist und aufeinander ausgerichtete Eingangs- und Ausgangslöcher aufweist, wobei dessen Distalende leicht verjüngt ist und dessen Proximalende wenigstens ein in bezug auf die Mittelachse des Knochennagels geneigtes Schraubenloch aufweist, dadurch gekennzeichnet, dass er an seinem Distalende (1b) vor dem verjüng-

ten Bereich wenigstens zwei Paar quer zur Achse (7) des Knochennagels (1) ausgerichtete Löcher (2, 3, 2a, 3a) aufweist, die den Knochennagel durchqueren, sowie Mittel (21, 21a) zum Positionieren und Verriegeln der Sonde besitzt, wobei die Positionierungsmittel je einem der Lochpaare entsprechen.

13. Knochennagel nach Anspruch 12, dadurch gekennzeichnet, dass die Positionierungsmittel durch eine besondere, von den Lochpaaren (2, 3, 2a, 3a) verschiedene Positionierunsbohrung (21, 21a) gebildet ist, die eine von der Sonde getragene, hervorstehende Kugel (20) aufnimmt.

14. Knochennagel, der in Verbindung mit der Sonde nach Anspruch 6 verwendbar ist, einen über den grössten Teil seiner Länge konstanten Querschnitt besitzt, und wenigstens im Distalendteil seiner Wand wenigstens ein Eingangsloch und wenigstens ein Ausgangsloch aufweist, das gemäss einer gemeinsamen Achse ausgerichtet ist, dadurch gekennzeichnet, dass die gemeinsame Achse (6) des Eingangsloches (2) und Ausgangsloches (3) zur Mittellängsachse (7) des Knochennagels (1) unter einem Winkel Alpha geneigt ist, der dem Neigungswinkel des Distalteils (17b) des Kanals (17) des Führungsblocks (15) der Sonde in bezug auf den Proximalteil (17a) dieses Kanals 17 gleich ist, und dass in der Wandung des Knochennagels eine mit der Positionierungskugel (20) des Führungsblocks (15) zusammenwirkende Positionierungsbohrung (21) vorgesehen ist in einem Abschnitt dieser Wand, der mit der Oberfläche des Knochennagels unmittelbar in Berührung kommt, wenn der Führungsblock (15) innerhalb des Knochennagels in den Bereich dieses Wandungsabschnitts eingeschoben wird.

15. Knochennagel nach Anspruch 14, dadurch gekennzeichnet, dass die Positionierungsausnehmung durch eine die Wand des Knochennagels 1 durchquerende Positionierungsbohrung (21) gebildet ist.

16. Knochennagel nach Anspruch 15, dadurch gekennzeichnet, dass die Achse der Positionierungsbohrung (21) in der gleichen Ebene liegt, wie die gemeinsame Achse (6) der Eingangs- und Auganslöcher (2, 3).

17. Knochennagel nach Anspruch 15 oder 16, dadurch gekennzeichnet, dass der Druckmesser der Positionierungbohrung (21) wesentlich grösser als der des Ausgangslochs (3) ist.

18. Knochennagel nach einem der Ansprüche 15 bis 17, dadurch gekennzeichnet, dass die Achse der Positionierungbohrung (21) in einer Ebene liegt, die in bezug auf die gemeinsame Achse (6) der Eingangs- und Ausgangslöcher (2, 3) winkelversetzt ist.

19. Knochennagel nach einem der Ansprüche 14 bis 18, dadurch gekennzeichnet, dass er eine Mehrzahl von Ausbuchtungen besitzt, während der Führungsblock ein Profil aufweist, das wenigstens einer dieser Ausbuchtungen angepasst ist.

20. Knochennagel nach Anspruch 19, dadurch gekennzeichnet, dass drei Ausbuchtungen (1a, 1b, 1c) vorgesehen sind, während das Profil des Führungsblocks (15) an zwei dieser Ausbuchtungen (1a, 1c) angepasst ist.

FIG.1

FIG.2

FIG. 3

FIG.4

A  B  C  D  E  F

EP 0 218 492 B1

FIG.6

FIG.5

FIG.7

FIG.8

FIG.9